(19) Europäisches Patentamt
European Patent Office
Office européen des brevets

(11) EP 0 697 395 B1

(12) # EUROPÄISCHE PATENTSCHRIFT

(45) Veröffentlichungstag und Bekanntmachung des Hinweises auf die Patenterteilung:
**07.04.1999 Patentblatt 1999/14**

(51) Int Cl.[6]: **C07C 209/16**

(21) Anmeldenummer: **95112481.7**

(22) Anmeldetag: **09.08.1995**

(54) **Verfahren zur Herstellung von Diaminen durch katalytische Aminierung von Aminoalkoholen**

Process for the preparation of diamines by catalytic amination of amino alcohols

Procédé de préparation de diamines par amination catalytique d'amino alcools

(84) Benannte Vertragsstaaten:
**BE CH DE ES FR GB IT LI**

(30) Priorität: **19.08.1994 DE 4429547**

(43) Veröffentlichungstag der Anmeldung:
**21.02.1996 Patentblatt 1996/08**

(73) Patentinhaber: **BASF AKTIENGESELLSCHAFT**
**67056 Ludwigshafen (DE)**

(72) Erfinder:
- **Becker, Rainer, Dr.**
  **D-67098 Bad Dürkheim (DE)**
- **Menger, Volkmar, Dr.**
  **D-67434 Neustadt (DE)**
- **Reif, Wolfgang, Dr.**
  **D-67227 Frankenthal (DE)**
- **Henne, Andreas, Dr.**
  **D-67433 Neustadt (DE)**

(56) Entgegenhaltungen:
**EP-A- 0 382 049** **EP-A- 0 514 692**
**EP-A- 0 696 572**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 697 395 B1

## Beschreibung

Die vorliegende Erfindung betrifft ein Verfahren zur Herstellung von Diaminen aus Aminoalkoholen und Stickstoffverbindungen in Gegenwart eines Zirkon-, Kupfer-, Nickelkatalysators bei Drücken von 1 bis 400 bar mit Wasserstoff.

Aus DE-A-19 53 263 ist es bekannt, Amine durch die hydrierende Aminierung der entsprechenden Alkohole an Cobalt, Nickel und Kupfer enthaltenden Katalysatoren herzustellen. Als Trägermaterial wird in diesen Katalysatoren Aluminium oxid oder Siliciumdioxid verwendet. Mit diesen Katalysatoren lassen sich bei hohen Temperaturen und Drücken gute Umsätze erzielen. Wird bei tieferen Temperaturen und Drücken gearbeitet, geht der Umsatz und die Selektivität stark zurück.

Aus der EP-A-254 335 sind Ni-Co-Ru-Katalysatoren auf Aluminiumoxid- oder Siliciumdioxid-Trägern, welche zusätzlich noch Halogenide in ihrer aktiven Masse enthalten, zur hydrierenden Aminierung von Alkoholen bekannt. Mit diesen Katalysatoren werden bei 200°C und 55 bar nur Umsätze von maximal 61 % erzielt.

Aus der US-A-4 151 204 sind Katalysatoren zur Herstellung von Aminoalkoholen bekannt, die aus einem Metall wie Cobalt, Nickel oder Kupfer, bevorzugt aus Nickel oder Cobalt bestehen, und gegebenenfalls mit geringen Mengen an Zirkonium dotiert sind, wobei das Zirkonium bezüglich des Nickels oder Cobalts in einem Mol-verhältnis von 0,005 : 1 bis 0,2 : 1 zugesetzt wird. Höhere Zirkoniumgehalte führen zu Nebenreaktionen wie der Zersetzung der Produkte.

Aus der EP-A-382 049 sind Katalysatoren und Verfahren zur hydrierenden Aminierung von Alkoholen bekannt. Diese Katalysatoren, deren aktive Masse sauerstoffhaltige Zirkon-, Kupfer-, Cobalt-und Nickelverbindungen enthält, zeichnen sich zwar durch eine gute Aktivität und Selektivität aus, besitzen allerdings verbesserungswürdige Standzeiten.

Die ältere europäische Anmeldung EP-A-696 572 offenbart die katalytische Aminierung von primären oder sekundären Alkoholen bei Temperaturen von 80 bis 250°C, Drücken von 1 bis 400 bar mit Wasserstoff und in Gegenwart eines Zirkon-, Kupfer-, Nickelkatalysators.

Der vorliegenden Erfindung lag daher die Aufgabe zugrunde, den zuvor genannten Nachteilen abzuhelfen.

Demgemäß wurde ein neues und verbessertes Verfahren zur Herstellung von Diaminen der allgemeinen Formel I

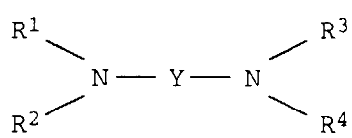 (I),

in der

$R^1$,$R^2$,$R^3$,$R^4$ Methyl, Ethyl, n-Propyl, n-Butyl und iso-Butyl,
$R^1$, $R^2$ zusätzlich Wasserstoff

und

Y eine gegebenenfalls durch $C_1$- bis $C_4$-Alkyl ein bis fünffach substituierte $C_2$- bis $C_{12}$-Alkylenkette

bedeuten, aus Aminoalkoholen der allgemeinen Formel II

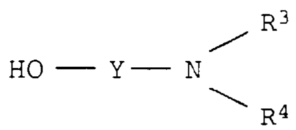 (II),

und Stickstoffverbindungen der allgemeinen Formel III

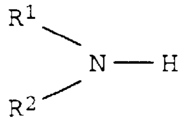

(III),

in denen $R^1$, $R^2$, $R^3$, $R^4$ und Y die oben genannten Bedeutungen haben, bei Drücken von 1 bis 400 bar mit Wasserstoff in Gegenwart eines Zirkon-, Kupfer-, Nickelkatalysators gefunden, welches dadurch gekennzeichnet ist, daß die katalytisch aktive Masse 20 bis 68,9 Gew.-% sauerstoffhaltige Zirkon-Verbindungen, berechnet als $ZrO_2$, 1 bis 30 Gew.-% sauerstoffhaltige Verbindungen des Kupfers, berechnet als CuO, 30 bis 70 Gew.-% sauerstoffhaltige Verbindungen des Nickels, berechnet als NiO, 0,1 bis 5 Gew.-% sauerstoffhaltige Verbindungen des Molybdäns, berechnet als $MoO_3$, und 0 bis 10 Gew.-% der sauerstoffhaltigen Verbindungen von Aluminium und/oder Mangan, berechnet als $Al_2O_3$ bzw. $MnO_2$, enthält und die Umsetzung bei Temperaturen von 155 bis 180°C durchgeführt wird.

In den Formeln I und II bedeutet Y

- eine gegebenenfalls durch $C_1$- bis $C_4$-Alkyl ein bis fünffach substituierte $C_2$- bis $C_{12}$-Alkylenkette wie $(CH_2)_2$, $(CH_2)_3$, $(CH_2)_4$, $(CH_2)_5$, $(CH_2)_6$, $(CH_2)_7$, $(CH_2)_8$, $(CH_2)_9$, $(CH_2)_{10}$, $(CH_2)_{11}$, $(CH_2)_{12}$, $CH(CH_3)CH_2$, $CH_2CH(CH_3)CH_2$, $CH_2CH_2CH(CH_3)$ und $CH_2CH_2CH_2CH(CH_3)$, bevorzugt eine gegebenenfalls durch Methyl oder Ethyl ein-bis dreifach substituierte $C_2$- bis $C_8$-Alkylenkette wie $(CH_2)_2$, $(CH_2)_3$, $(CH_2)_4$, $(CH_2)_5$, $(CH_2)_8$, $(CH_2)_7$, $(CH_2)_8$, $CH(CH_3)CH_2$, $CH_2CH(CH_3)CH_2$, $CH_2CH_2CH(CH_3)$ und $CH_2CH_2CH_2CH(CH_3)$, besonders bevorzugt eine gegebenenfalls durch Methyl ein-bis dreifach substituierte $C_2$- bis $C_6$-Alkylenkette wie $(CH_2)_2$, $(CH_2)_3$, $(CH_2)_4$, $(CH_2)_5$, $(CH_2)_8$, $CH(CH_3)CH_2$, $CH_2CH(CH_3)CH_2$, $CH_2CH_2CH(CH_3)$ und $CH_2CH_2CH_2CH(CH_3)$.

Bevorzugt werden beispielsweise die folgenden Aminoalkohole aminiert:

N,N-Dimethylaminoethanol, N,N-Diethylamino-ethanol, N,N-Di-n-propylaminoethanol, N,N-Di-n-butylaminoetnanol, N,N-Di-iso-butyl-aminoethanol, N,N -Dimethylaminopropanol, N,N-Diethylaminopropanol, N,N-Di-n-propylaminopropanol, N,N-Di-n-butylaminopropanol, N,N-Di-iso-butylaminopropanol, Dimethylaminopentanol-4 und Diethylaminopentanol-4.

Als Aminierungsmittel bei der hydrierenden Aminierung von Aminoalkoholen können sowohl Ammoniak als auch primäre oder sekundäre aliphatische Amine eingesetzt werden.

Bei Verwendung von Ammoniak als Aminierungsmittel werden die alkoholischen Hydroxylgruppen zunächst in freie Aminogruppen ($-NH_2$) umgewandelt. Die so gebildeten primären Amine können mit weiterem Alkohol zu den entsprechenden sekundären Aminen und diese wiederum mit weiterem Alkohol zu den entsprechenden symmetrischen tertiären Aminen reagieren. Je nach Zusammensetzung des Reaktionsansatzes und je nach den angewandten Reaktionsbedingungen - Druck, Temperatur, Reaktionszeit - lassen sich auf diese Weise je nach Wunsch bevorzugt primäre, sekundäre oder tertiäre Amine darstellen.

Ebenso wie Ammoniak lassen sich primäre oder sekundäre Amine als Aminierungsmittel verwenden.

Bevorzugt werden beispielsweise die folgenden Mono- und Dialkylamine als Aminierungsmittel verwendet: Methylamin, Dimethylamin, Ethylamin, Diethylamin, Propylamin und Butylamin.

Das Aminierungsmittel kann bezüglich der zu aminierenden alkoholischen Hydroxylgruppe in stöchiometrischer Menge eingesetzt werden. Bevorzugt wird jedoch mit einem Überschuß an Aminierungsmittel gearbeitet und zwar im allgemeinen mit einem mehr als 5 molaren Überschuß pro Mol zu aminierender alkoholischer Hydroxylgruppe. Speziell Ammoniak wird im allgemeinen mit einem 1,5 bis 250-fachen, bevorzugt 5 bis 100-fachen, insbesondere 10 bis 40-fachen molaren Überschuß pro Mol umzusetzender alkoholischer Hydroxylgruppen eingesetzt. Höhere Überschüsse sowohl an Ammoniak als auch an primären oder sekundären Aminen sind möglich.

Der Wasserstoff wird der Reaktion im allgemeinen in einer Menge von 5 bis 400Nl, bevorzugt in einer Menge von 50 bis 200N1 pro Mol Alkoholkomponente zugeführt.

Die Umsetzung erfolgt im allgemeinen ohne zusätzliches Lösungsmittel. Bei der Umsetzung hochmolekularer, hochviskoser oder bei Raumtemperatur fester Ausgangsverbindungen oder Produkte kann es vorteilhaft sein, ein unter den Reaktionsbedingungen inertes Lösungsmittel, wie Tetrahydrofuran, Dioxan, N-Methylpyrrolidon oder Ethylenglykoldimethylether mitzuverwenden.

Üblicherweise arbeitet man bei der Umsetzung bei Temperaturen von 155 bis 250°C, bevorzugt 155 bis 200°C. Im allgemeinen wird die Reaktion bei einem Druck von 1 bis 400 bar ausgeführt. Bevorzugt werden jedoch Drücke von 10 bis 250 bar, insbesondere von 30 bis 220 bar angewandt.

Die Anwendung höherer Temperaturen und eines höheren Gesamtdruckes ist möglich. Der Gesamtdruck im Reaktionsgefäß, welches sich aus der Summe der Partialdrücke des Aminierungsmittels, der Alkoholkomponente und der gebildeten Reaktionsprodukte sowie ggf. des mitverwendeten Lösungsmittels bei den angegebenen Temperaturen ergibt, wird zweckmäßigerweise durch Aufpressen von Wasserstoff auf den gewünschten Reaktionsdruck eingestellt.

Es kann für die Selektivität des vorliegenden Verfahrens vorteil-haft sein, die Katalysatorformkörper im Reaktor mit inerten Füllkörpern zu vermischen, sie sozusagen zu "verdünnen". Der Anteil der Füllkörper in solchen Katalysatorzubereitungen kann 20 bis 80, besonders 30 bis 60 und insbesonders 40 bis 50 Volumenteile betragen.

Praktisch geht man bei der Durchführung im allgemeinen so vor, daß man dem Katalysator, der sich üblicherweise in einem bevorzugt von außen beheizten Festbettreaktor befindet, bei der gewünschten Reaktionstemperatur und dem gewünschten Druck den Alkohol und das Aminierungsmittel simultan zuführt. Dabei belastet man den Katalysator im allgemeinen mit 0,02 bis 3, bevorzugt 0,05 bis 2 und besonders bevorzugt mit 0,1 bis 1,61 Alkohol pro Liter Katalysator und Stunde. Hierbei ist es zweckmäßig, die Reaktanten bereits vor der Zuführung in das Reaktionsgefäß zu erwärmen und zwar bevorzugt auf die Reaktionstemperatur.

Der Reaktor kann sowohl in der Sumpf- als auch in der Rieselfahrweise betrieben werden, d.h. man kann die Reaktanten sowohl von unten nach oben als auch von oben nach unten durch den Reaktor leiten. Es versteht sich von selbst, daß sich das Verfahren sowohl diskontinuierlich als auch kontinuierlich durchführen läßt. In beiden Fällen kann das überschüssige Aminierungsmittel zusammen mit dem Wasserstoff im Kreis geführt werden. Ist der Umsatz bei der Reaktion nicht vollständig, so kann das nicht umgesetzte Ausgangsmaterial ebenfalls in die Reaktionszone zurückgeführt werden.

Aus dem Reaktionsaustrag werden, nachdem dieser zweckmäßigerweise entspannt worden ist, das überschüssige Aminierungsmittel und der Wasserstoff entfernt und die erhaltenen aminierten Produkte durch Destillation, Flüssigextraktion oder Kristallisation gereinigt. Das überschüssige Aminierungsmittel und der Wasserstoff werden vorteilhaft wieder in die Reaktionszone zurückgeführt. Das gleiche gilt für die eventuell nicht oder nicht vollständig umgesetzte Alkoholkomponente.

Das im Zuge der Umsetzung gebildete Reaktionswasser wirkt sich im allgemeinen auf den Umsetzungsgrad, die Reaktionsgeschwindigkeit, die Selektivität und die Katalysatorstandzeit nicht störend aus und wird deshalb zweckmäßigerweise erst bei der destillativen Aufarbeitung des Reaktionsproduktes aus diesem entfernt.

Im allgemeinen werden die erfindungsgemäßen Katalysatoren bevorzugt in Form von Vollkatalysatoren eingesetzt. Mit dem Begriff "Vollkatalysator" wird ein Katalysator bezeichnet, der im Gegensatz zu einem Trägerkatalysator nur aus katalytisch aktiver Masse besteht. Vollkatalysatoren können dergestalt eingesetzt werden, daß man die katalytisch aktive, zu Pulver vermahlene Masse in das Reaktionsgefäß einbringt oder, daß man die katalytisch aktive Masse nach Mahlung, Vermischung mit Formhilfsmitteln, Formung und Temperung als Katalysatorformkörper - beispielsweise als Kugeln, Zylinder, Ringe, Spiralen - im Reaktor anordnet.

Die katalytisch aktive Masse der erfindungsgemäßen Katalysatoren enthält neben sauerstoffhaltigen Verbindungen des Zirkoniums, sauerstoffhaltige Verbindungen des Nickels, Kupfers und Molybdäns.

Da sich die Konzentrationsangaben jeweils - falls nicht anders angegeben - auf die katalytisch aktive Masse des Katalysators beziehen, wird die katalytisch aktive Masse des Katalysators im folgenden als die Summe der Massen der katalytisch aktiven Bestandteile Zirkonium, Nickel, Kupfer und Molybdän im Katalysator, jeweils berechnet als $ZrO_2$, NiO, CuO bzw. $MoO_3$, nach dessen letzter Wärmebehandlung und vor dessen Reduktion mit Wasserstoff, definiert.

Im allgemeinen beträgt der Zirkoniumoxidgehalt der erfindungsgemäßen Katalysatoren zwischen 20 und 68,9, bevorzugt 25 bis 49,5 Gew.-%.

Die anderen Komponenten Nickel, Kupfer und Molybdän sind im allgemeinen insgesamt in Mengen von 31,1 bis 80, bevorzugt 50,5 bis 70 Gew.-%, in der katalytisch aktiven Masse enthalten.

Bevorzugte Katalysatoren enthalten in ihrer katalytisch aktiven Masse 20 bis 68,9 Gew.-%, bevorzugt 25 bis 49,5 Gew.-%, sauerstoffhaltige Zirkoniumverbindungen, 1 bis 30 Gew.-%, bevorzugt 10 bis 25 Gew.-% sauerstoffhaltige Kupferverbindungen, 30 bis 70 Gew.-%, bevorzugt 40 bis 64,5 Gew.-%, sauerstoffhaltige Verbindungen des Nickels, 0,1 bis 5 Gew.-%, bevorzugt 0,5 bis 3,5 Gew.-% sauerstoffhaltige Verbindungen des Molybdäns und 0 bis 10 Gew.-% sauerstoffhaltige Verbindungen von Aluminium und/oder Mangan.

Zur Herstellung der Vollkatalysatoren sind verschiedenerlei Verfahrensweisen möglich. Sie sind beispielsweise durch Anteigen pulvriger Mischungen der Hydroxide, Carbonate, Oxide und/oder anderer Salze der Komponenten Zirkonium, Cobalt, Nickel und Kupfer mit Wasser und nachfolgendes Extrudieren und Tempern der so erhaltenen Masse erhältlich.

Im allgemeinen werden zur Herstellung der erfindungsgemäßen Katalysatoren jedoch Fällungsmethoden angewandt. So können sie beispielsweise durch eine gemeinsame Fällung der Cobalt-, Nickelund Kupferkomponenten aus einer diese Elemente enthaltenden, wäßrigen Salzlösung mittels Mineralbasen in Gegenwart einer Aufschlämmung einer schwerlöslichen, sauerstoffhaltigen Zirkoniumverbindung und anschließendes Waschen, Trocknen und Calcinieren des erhaltenen Präzipitats erhalten werden. Als schwerlösliche, sauerstoffhaltige Zirkoniumverbindungen können beispielsweise Zirkoniumdioxid, Zirkoniumoxidhydrat, Zirkoniumphosphate, -borate und -silikate Verwendung finden. Die Aufschlämmungen der schwerlöslichen Zirkoniumverbindungen können durch Suspendieren feinkörniger Pulver dieser Verbindungen in Wasser unter kräftigem Rühren hergestellt werden, vorteilhaft werden diese Aufschlämmungen durch Ausfällen der schwerlöslichen Zirkoniumverbindungen aus wäßrigen Zirkoniumsalzlösungen mittels Mineralbasen erhalten.

Bevorzugt werden die erfindungsgemäßen Katalysatoren über eine gemeinsame Fällung (Mischfällung) aller ihrer Komponenten hergestellt. Dazu wird zweckmäßigerweise eine die Katalysatorkomponenten enthaltende, wäßrige Salzlösung in der Wärme und unter Rühren so lange mit einer wäßrigen Mineralbase, insbesondere einer Alkalimetallbase - beispielsweise Natriumcarbonat, Natriumhydroxid, Kaliumcarbonat oder Kaliumhydroxid - versetzt, bis die Fällung vollständig ist. Die Art der verwendeten Salze ist im allgemeinen nicht kritisch - da es bei dieser Vorgehensweise vornehmlich auf die Wasserlöslichkeit der Salze ankommt, ist ein Kriterium ihre zur Herstellung dieser verhältnismäßig stark konzentrierten Salzlösungen erforderlich, gute Wasserlöslichkeit. Es wird als selbstverständlich erachtet, daß bei der Auswahl der Salze der einzelnen Komponenten natürlich nur Salze mit solchen Anionen gewählt werden, die nicht zu Störungen führen, sei es, indem sie unerwünschte Fällungen verursachen oder indem sie durch Komplexbildung die Fällung erschweren oder verhindern.

Erfindungsgemäße Katalysatoren mit besonders vorteilhaften Eigenschaften sind dadurch erhältlich, daß man einen Teil der Zirkoniumkomponente des Katalysators, zweckmäßigerweise aus einer wäßrigen Zirkoniumsalzlösung, separat in einer Fällungsapparatur durch Zugabe wäßriger Mineralbasen fällt. Auf das so erhaltene, vorzugsweise frisch gefällte Zirkoniumoxid-Hydrat, kann dann der restliche Teil der Zirkoniumkomponente des Katalysators zusammen mit den anderen katalytisch aktiven Komponenten in einer Mischfällung gefällt werden, wie oben beschrieben wurde. Dabei erweist es sich in der Regel als besonders zweckmäßig, 10 bis 80 Gew.-%, vorzugsweise 30 bis 70 Gew.-% und insbesondere 40 bis 60 Gew.-% der Gesamtzirkoniummenge der katalytisch aktiven Masse vorzufällen.

Die bei diesen Fällungsreaktionen enthaltenen Niederschläge sind im allgemeinen chemisch uneinheitlich und bestehen u.a. aus Mischungen der Oxide, Oxidhydrate, Hydroxide, Carbonate und unlöslichen und basischen Salze der genannten Metalle. Es kann sich für die Filtrierbarkeit der Niederschläge als günstig erweisen, wenn sie gealtert werden, d.h. wenn man sie noch einige Zeit nach der Fällung, gegebenenfalls in Wärme oder unter Durchleiten von Luft, sich selbst überläßt.

Die nach diesen Fällungsverfahren erhaltenen Niederschläge werden wie üblich zu den erfindungsgemäßen Katalysatoren weiterverarbeitet. Nach dem Waschen werden sie im allgemeinen bei 80 bis 200°C, vorzugsweise bei 100 bis 150°C, getrocknet und danach calciniert. Die Calcinierung wird im allgemeinen bei Temperaturen zwischen 300 und 800°C, vorzugsweise bei 400 bis 600°C, insbesondere bei 450 bis 550°C ausgeführt.

Nach der Calcinierung wird der Katalysator zweckmäßigerweise konditioniert, sei es, daß man ihn durch Vermahlen auf eine bestimmte Korngröße einstellt oder daß man ihn nach seiner Vermahlung mit Formhilfsmitteln wie Graphit oder Stearinsäure vermischt, mittels einer Tablettenpresse zu Formlingen verpreßt und tempert. Die Tempertemperaturen entsprechen dabei im allgemeinen den Temperaturen bei der Calcinierung.

Die auf diese Weise hergestellten Katalysatoren enthalten die katalytisch aktiven Metalle in Form eines Gemisches ihrer sauerstoffhaltigen Verbindungen, d.h. insbesondere als Oxide und Mischoxide.

Die auf diese Weise hergestellten Katalysatoren werden als solche gelagert und ggf. gehandelt. Vor ihrem Einsatz als Katalysatoren zur hydrierenden Aminierung von Alkoholen werden sie üblicherweise vorreduziert. Sie können jedoch auch ohne Vorreduktion eingesetzt werden, wobei sie dann unter den Bedingungen der hydrierenden Aminierung durch den im Reaktor vorhandenen Wasserstoff reduziert werden. Zur Vorreduktion werden die Katalysatoren im allgemeinen zunächst bei 150 bis 200°C über einen Zeitraum von 12 bis 20 Stunden einer Stickstoff-Wasserstoff-Atmosphäre ausgesetzt und anschließend noch bis zu ca. 24 Stunden bei 200 bis 300°C in einer Wasserstoffatmosphäre behandelt. Bei dieser Vorreduktion wird ein Teil der in den Katalysatoren vorliegenden sauerstoffhaltigen Metallverbindungen zu den entsprechenden Metallen reduziert, so daß diese gemeinsam mit den verschiedenartigen Sauerstoffverbindungen in der aktiven Form des Katalysators vorliegen.

Die erfindungsgemäß erhältlichen Diamine eignen sich u.a. als Zwischenprodukte bei der Herstellung von Kraftstoffadditiven (US-A-3 275 554; DE-A-21 25 039 und DE-A-36 11 230), Tensiden, Arznei- und Pflanzenschutzmitteln sowie von Vulkanisationsbeschleunigern.

Beispiele

Katalysatorherstellung

Herstellung von Katalysator A

Eine wäßrige Lösung aus Nickelnitrat, Kupfernitrat und Zirkonacetat, die 4,48 % NiO, 1,52 % CuO und 2,82 % $ZrO_2$ enthielt, wurde gleichzeitig in einem Rührgefäß in einem konstanten Strom mit einer 20 %igen wäßrigen Natriumcarbonatlösung bei einer Temperatur von 70°C so gefällt, daß der mit einer Glaselektrode gemessene pH-Wert von 7,0 aufrechterhalten wurde.

Die erhaltene Suspension wurde filtriert und der Filterkuchen mit vollentsalztem Wasser gewaschen, bis die elektrische Leitfähigkeit des Filtrats ca. 20 µS betrug. Dann wurde in den noch feuchten Filterkuchen so viel Ammoniumheptamolybdat eingearbeitet, daß das nachfolgend angegebene Oxidgemisch erhalten wurde. Danach wurde der

Filterkuchen bei einer Temperatur von 150°C in einem Trockenschrank oder einem Sprühtrockner getrocknet. Das auf diese Weise erhaltene Hydroxidcarbonatgemisch wurde nun bei einer Temperatur von 500°C über einen Zeitraum von 4 Stunden getempert.

Der so erhaltene Katalysator hatte die Zusammensetzung: 50 Gew.-% NiO, 17 Gew.-% CuO, 1,5 Gew.-% $MoO_3$ und 31,5 Gew.-% $ZrO_2$. Das Katalysatorpulver wurde mit 3 Gew.-% Graphit vermischt und zu 6 x 3 mm-Tabletten verformt. Die Tabletten hatten eine Porosität (gemessen über die Wasseraufnahme) von 0,20 ml/g und eine Härte von 3500 N/$cm^2$.

Beispiel 1

Aminierung von 1-Diethylaminopentanol-4

Ein kontinuierlich betriebener Hochdruckreaktor wurde mit 700 ml des Katalysators A beschickt. Zur Aminierung des Diethylaminopentanol-4 werden die in nachstehender Tabelle angegebenen Bedingungen eingestellt und die ebenfalls notierten Werte der Rohaustragsanalysen erhalten (Druck konstant 200 bar)

| Temp. [°C] | Belastung*) | Mol-Verhältnis Alkohol:$NH_3$ | Umsatz [%] | Selektivität**) [%] |
|---|---|---|---|---|
| 180 | 0,42 | 1:20 | 97 | 80 |
| 180 | 0,84 | 1:20 | 91 | 90 |
| 170 | 0,42 | 1:20 | 84 | 90 |
| 170 | 0,62 | 1:20 | 68 | 93 |
| 160 | 0,31 | 1:20 | 70 | 94 |
| 160 | 0,2 | 1:20 | 73 | 94 |
| 165 | 0,2 | 1:20 | 78 | 90 |
| 165 | 0,2 | 1:10 | 68 | 82 |
| 165 | 0,2 | 1:30 | 82 | 91 |
| 165 | 0,2 | 1:40 | 83 | 92 |
| 165 | 0,2 | 1:5 | 65 | 63 |

*) Belastung: kg Alkohol/l Kat·h

**) Selektivität = $\frac{\text{Ausbeute Diethylaminopentylamin}}{\text{Umsatz Diethylaminopentanol}} \cdot 100$

Die Ergebnisse zeigen, daß unter geeigneten Bedingungen Selektivitäten >90 % zu erreichen sind. Der Versuch wird über insgesamt 150 Tage betrieben; der Katalysator zeigt nach der Versuchsperiode beim Ausbau keine Zerfallserscheinungen.

Beispiel 2

Scale-up-Versuch zur Aminierung von 1-Diethylaminopentanol-4

Ein kontinuierlich betriebener Hochdruckreaktor wird mit 60 l Katalysator (Typ A) gefüllt und mit stündlich 15 l Diethylaminopentanol und 40 l Ammoniak (entspricht Molverhältnis 1:20) bei 200 bar und 155-157°C in Sumpffahrweise beschickt. Der Ofenaustrag zeigt bei einem Umsatz von 80 % eine Wertproduktselektivität von 91 %.

## Patentansprüche

1. Verfahren zur Herstellung von Diaminen der allgemeinen Formel I

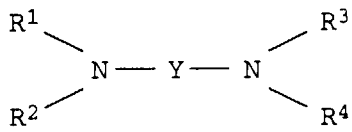 (I),

in der

$R^1,R^2,R^3,R^4$ Methyl, Ethyl, n-Propyl, n-Butyl und iso-Butyl,
$R^1$, $R^2$ zusätzlich Wasserstoff

und

Y eine gegebenenfalls durch $C_1$- bis $C_4$-Alkyl ein bis fünffach substituierte $C_2$- bis $C_{12}$-Alkylenkette

bedeuten, aus Aminoalkoholen der allgemeinen Formel II

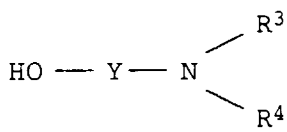 (II),

und Stickstoffverbindungen der allgemeinen Formel III

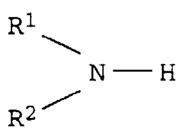 (III),

in denen $R^1$, $R^2$, $R^3$, $R^4$ und Y die oben genannten Bedeutungen haben, bei Drücken von 1 bis 400 bar mit Wasserstoff in Gegenwart eines Zirkon-, Kupfer-, Nickelkatalysators, dadurch gekennzeichnet, daß die katalytisch aktive Masse 20 bis 68,9 Gew.-% sauerstoffhaltige Zirkon-Verbindungen, berechnet als ZrO2, 1 bis 30 Gew.-% sauerstoffhaltige Verbindungen des Kupfers, berechnet als CuO, 30 bis 70 Gew.-% sauerstoffhaltige Verbindungen des Nickels, berechnet als NiO, 0,1 bis 5 Gew.-% sauerstoffhaltige Verbindungen des Molybdäns, berechnet als $MoO_3$ und 0 bis 10 Gew.-% der sauerstoffhaltigen Verbindungen von Aluminium und/oder Mangan, berechnet als $Al_2O_3$ bzw. $MnO_2$, enthält und die Umsetzung bei Temperaturen von 155 bis 180°C durchgeführt wird.

**2.** Verfahren nach Anspruch 1, dadurch gekennzeichnet, daß die Umsetzung bei Temperaturen von 155-170°C und einem molaren Verhältnis von Ammoniak zu Aminoalkohol von 10 bis 40 durchgeführt wird.

**3.** Verfahren nach Anspruch 1, dadurch gekennzeichnet, daß die katalytisch aktive Masse 40 bis 64,5 Gew.-% sauerstoffhaltige Verbindungen des Nickels, berechnet als NiO, enthält.

**4.** Verfahren nach Anspruch 1, dadurch gekennzeichnet, daß die katalytisch aktive Masse 0,5 bis 3,5 Gew.-% sauerstoffhaltige Verbindungen des Molybdäns, berechnet als $MoO_3$, enthält.

**5.** Verfahren nach Anspruch 1, dadurch gekennzeichnet, daß die katalytisch aktive Masse 25 bis 49,5 Gew.-% sauerstoffhaltige Zirkon-Verbindungen, berechnet als $ZrO_2$, enthält.

**6.** Verfahren nach Anspruch 1, dadurch gekennzeichnet, daß die katalytisch aktive Masse 10 bis 25 Gew.-% sauerstoffhaltige Verbindungen des Kupfers, berechnet als CuO, enthält.

**7.** Verfahren nach Anspruch 1, dadurch gekennzeichnet, daß man die Umsetzung bei Drücken von 10 bis 250 bar durchführt.

**8.** Verfahren nach Anspruch 1, dadurch gekennzeichnet, daß man die Umsetzung bei Drücken von 30 bis 220 bar durchführt.

**Claims**

**1.** A process for preparing diamines of the formula I

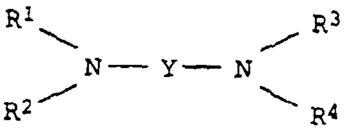

(I),

where

| | |
|---|---|
| $R^1$, $R^2$, $R^3$, $R^4$ | are methyl, ethyl, n-propyl, n-butyl or iso-butyl, |
| $R^1$, $R^2$ | may also be hydrogen |

and

Y is a $C_2$-$C_{12}$-alkylene chain which is unsubstituted or monosubstituted to pentasubstituted by $C_1$-$C_4$-alkyl,

from aminoalcohols of the formula (II)

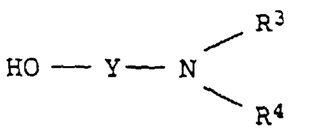

(II),

and nitrogen compounds of the formula (III)

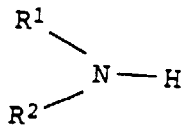

(III),

where $R^1$, $R^2$, $R^3$, $R^4$ and Y are as defined above, at pressures of from 1 to 400 bar using hydrogen in the presence of a zirconium, copper and nickel catalyst, wherein the catalytically active composition comprises from 20 to 68.9% by weight of oxygen-containing zirconium compounds, calculated as $ZrO_2$, from 1 to 30% by weight of oxygen-containing compounds of copper, calculated as CuO, from 30 to 70% by weight of oxygen-containing compounds of nickel, calculated as NiO, from 0.1 to 5% by weight of oxygen-containing compounds of molybdenum, calculated as $MoO_3$, and from 0 to 10% by weight of oxygen-containing compounds of aluminum and/or manganese, calculated as $Al_2O_3$ and $MnO_2$, and the reaction is carried out at from 155 to 180°C.

**2.** A process as claimed in claim 1, wherein the reaction is carried out at 155-170°C and a molar ratio of ammonia to aminoalcohol of from 10 to 40.

**3.** A process as claimed in claim 1, wherein the catalytically active composition contains from 40 to 64.5% by weight

of oxygen-containing compounds of nickel, calculated as NiO.

**4.** A process as claimed in claim 1, wherein the catalytically active composition contains from 0.5 to 3.5% by weight of oxygen-containing compounds of molybdenum, calculated as $MoO_3$.

**5.** A process as claimed in claim 1, wherein the catalytically active composition contains from 25 to 49.5% by weight of oxygen-containing zirconium compounds, calculated as ZrO2.

**6.** A process as claimed in claim 1, wherein the catalytically active composition contains from 10 to 25% by weight of oxygen-containing compounds of copper, calculated as CuO.

**7.** A process as claimed in claim 1, wherein the reaction is carried out at pressures of from 10 to 250 bar.

**8.** A process as claimed in claim 1, wherein the reaction is carried out at pressures of from 30 to 220 bar.

**Revendications**

**1.** Procédé de préparation de diamines de formule générale I

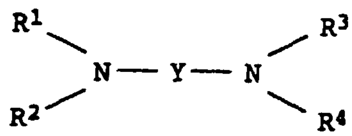

(I),

dans laquelle

$R^1$, $R^2$, $R^3$, $R^4$ sont des groupes méthyle, éthyle, n-propyle, n-butyle et isobutyle,
$R^1$ et $R^2$ représentent en outre un atome d'hydrogène,

et

Y est une chaîne alkylène en $C_2$ à $C_{12}$, éventuellement une à cinq fois substituée par des substituants alkyle en $C_1$ à $C_4$,

à partir d'amino-alcools de formule générale II

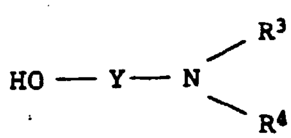

(II),

et de composés azotés de formule générale III

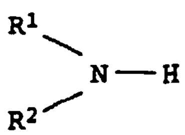

(III),

dans lesquelles $R^1$, $R^2$, $R^3$, $R^4$ et Y ont les significations données ci-dessus, sous des pressions de 1 à 400 bar, avec de l'hydrogène en présence d'un catalyseur au zirconium, au cuivre, au nickel, caractérisé en ce que la masse

catalytiquement active contient de 20 à 68,9 % en poids de composés oxygénés du zirconium, exprimés en $ZrO_2$, de 1 à 30 % en poids de composés oxygénés du cuivre, exprimés en CuO, de 30 à 70 % en poids de composés oxygénés du nickel, exprimés en NiO, de 0,1 à 5 % en poids de composés oxygénés du molybdène, exprimés en $MoO_3$ et de 0 à 10 % en poids de composés oxygénés de l'aluminium et/ou du manganèse, exprimés respectivement en $Al_2O_3$ et $MnO_2$, la réaction étant mise en oeuvre à des températures de 155 à 180°C.

**2.** Procédé selon la revendication 1, caractérisé en ce que la réaction est mise en oeuvre à des températures de 155 à 170°C, pour un rapport en moles de l'ammoniac à l'amino-alcool de 10 à 40.

**3.** Procédé selon la revendication 1, caractérisé en ce que la masse catalytiquement active contient de 40 à 64,5 % en poids de composés oxygénés du nickel, exprimés en NiO.

**4.** Procédé selon la revendication 1, caractérisé en ce que la masse catalytiquement active contient de 0,5 à 3,5 % en poids de composés oxygénés du molybdène, exprimés en $MoO_3$.

**5.** Procédé selon la revendication 1, caractérisé en ce que la masse catalytiquement active contient de 25 à 49,5 % en poids de composés oxygénés du zirconium, exprimés en $ZrO_2$.

**6.** Procédé selon la revendication 1, caractérisé en ce que la masse catalytiquement active contient de 10 à 25 % en poids de composés oxygénés du cuivre, exprimés en CuO.

**7.** Procédé selon la revendication 1, caractérisé en ce qu'on met en oeuvre la réaction sous des pressions de 10 à 250 bar.

**8.** Procédé selon la revendication 1, caractérisé en ce qu'on met en oeuvre la réaction sous des pressions de 30 à 220 bar.